# EUROPÄISCHE PATENTSCHRIFT

(11) **EP 1 607 090 B1**
(45) Veröffentlichungstag und Bekanntmachung des Hinweises auf die Patenterteilung: **12.10.2011**
(21) Anmeldenummer: 05016750.1
(22) Anmeldetag: 16.04.1999
(51) Int. Cl.: A61K 31/40, A61P 1/00

(54) **Asimadolin für die Behandlung des Irritable Bowel Syndroms**
Asimadoline for the treatment of irritable bowel syndrome
Asimadoline pour le traitement du syndrome du colon irritable

(30) Priorität: 20.04.1998 DE 19817393; 20.06.1998 DE 19827633
(43) Veröffentlichungstag der Anmeldung: 21.12.2005
(62) Teilanmeldung aus: 99923421.4
(73) Patentinhaber: Tioga Pharmaceuticals, Inc., San Diego, CA 92121 (US)
(72) Erfinder: Bathe, Andreas, Dr., 64283 Darmstadt (DE); Helfert, Bernd, 64372 Ober-Ramstadt (DE); Ackermann, Karl-August, 64372 Ober-Ramstadt (DE); Gottschlich, Rudolf, Dr., 64354 Reinheim (DE); Stein, Ingeborg, Dr., 63110 Rodgau (DE); Budak, Jens, 64287 Darmstadt (DE)
(74) Vertreter: Viering, Jentschura & Partner

(56) Entgegenhaltungen:
- EP-A- 0 752 246
- WO-A-98/03491
- BARBER A ET AL: "A PHARMACOLOGICAL PROFILE OF THE NOVEL, PERIPHERALLY-SELECTIVE KAPPA-OPIOID RECEPTOR AGONIST, EMD 61753" BRITISH JOURNAL OF PHARMACOLOGY, BASINGSTOKE, HANTS, GB, Bd. 113, Nr. 4, 1. Dezember 1994 (1994-12-01), Seiten 1317-1327, XP000196255 ISSN: 0007-1188
- FRIEDMAN L S ET AL: "IRRITABLE BOWEL SYNDROME" MOUNT SINAI JOURNAL OF MEDICINE, NEW YORK, NY, US, Bd. 63, Nr. 2, März 1996 (1996-03), Seiten 126-133, XP009023687 ISSN: 0027-2507
- DATABASE BIOSIS [Online] BIOSCIENCES INFORMATION SERVICE, PHILADELPHIA, PA, US; 1995, DAPOIGNY M ET AL: "Efficacy of peripheral kappa agonist fedotozine versus placebo in treatment of irritable bowel syndrome: A multicenter dose-response study" XP002347915 Database accession no. PREV199698611455 & DIGESTIVE DISEASES AND SCIENCES, Bd. 40, Nr. 10, 1995, Seiten 2244-2249, ISSN: 0163-2116

## Beschreibung

Die Erfindung betrifft die Verwendung von N-Methyl-N-[(1S)-1-phenyl-2-((3S)-3-hydroxypyrrolidin-1-yl)-ethyl]-2,2-diphenylacetamid und/oder eines physiologisch verträglichen Salzes davon zur Herstellung eines Arzneimittels zur Behandlung von Irritable Bowel Syndrome (IBS).

Wie von Barber et al. (B. J. Pharmacol. (1994), **113**, 1317-1327) beschrieben, besitzen sowohl die Verbindung N-Methyl-N-[(1S)-1-phenyl-2-((3S)-3-hydroxypyrrolidin-1-yl)-ethyl]-2,2-diphenylacetamid, als auch ihre physiologisch verträglichen Salze wertvolle pharmakologische Eigenschaften wie eine analgetische, antiinflammatorische und aquaretische Wirkung, so daß sie besonders zur Herstellung von Arzneimitteln geeignet sind.

Es wurde, wie in der Patentanmeldung DE 1 95 23 502 bzw. EP 752 246 beschrieben, gefunden, daß diese Verbindung eine besonders wirksame Verbindung ist, die sich als Arzneimittel zur Behandlung entzündlicher Darmerkrankungen in ganz besonderer Weise eignet. Insbesondere ist diese Verbindung bei dieser Indikation einsetzbar und wirksam, da sie gleichzeitig die mit dieser Erkrankung verbundenen Schmerzen lindert und im akuten Fall eines durch die entzündliche Darmerkrankung drohenden bzw. hervorgerufenen Darmverschlusses die Motorik des Darms wieder normalisiert oder wieder in Gang setzt, ohne spürbare Nebenwirkungen hervorzurufen. Außerdem kann die Verbindung bei nicht-entzündlichen Darmerkrankungen wie IBS (Irritable Bowel Syndrome) eingesetzt werden.

In den Patentanmeldungen DE 40 34 785 A1 und DE 42 15 213 A1 bzw. EP 0 569 802 A1 ist die Herstellung von N-Methyl-N-[(1S)-1-phenyl-2-((3S)-3-hydroxypyrrolidin-1-yl)-ethyl]-2,2-diphenylacetamid durch Umsetzung von (2S)-2-N-Carboxyethyl-2-phenylglycin-N,N-[(3S)-3-hydroxy-tetramethylamid mit Diphenylacetylchlorid beschrieben. Wie in DE 42 15 213 beschrieben, ist die Ausgangsverbindung (2S)-2-N-Carboxyethyl-2-phenylglycin-N,N-[(3S)-3-hydroxytetramethylenamid, auch als (1 S)-[1-N-Methyl-amino-1-phenyl-2-((3S)-3-hydroxy-pyrrolidino)ethan benannt, herstellbar, indem (1S)-1-Amino-1-phenyl-2-chlorethan mit (3S)-3-Hydroxy-pyrrolidin umgesetzt und anschließend mit Methyljodid methyliert wird. Probleme dieser Herstellungsmethode bestehen jedoch in der Löslichkeit der Ausgangsprodukte und darin, daß im Anschluß an die Synthese das erhaltene, durch Nebenprodukte verunreinigte racemische Produktgemisch aufwendig aufgetrennt werden muß. Das bisher bekannte Verfahren zur Herstellung von N-Methyl-N-[(1S)-1-phenyl-2-((3S)-3-hydroxypyrrolidin-1-yl)-ethyl]-2,2-diphenylacetamid ist daher aufwendig und teuer und führt zu geringen Ausbeuten bezogen auf die eingesetzten Ausgangsverbindungen.

Hierin beschrieben wird ein in einfacher Weise durchführbares und preiswertes Verfahren zur Herstellung von N-Methyl-N-[(1S)-1-phenyl-2-((3S)-3-hydroxypyrrolidin-1-yl)-ethyl]-2,2-diphenylacetamid bzw. bei Einsatz der enantiomeren Edukte von N-Methyl-N-[(1 R)-1-phenyl-2-((3R)-3-hydroxypyrrolidin-1-yl)-ethyl]-2,2-diphenylacetamid, das von preiswerten, gut löslichen Ausgangsprodukten ausgeht, die zu einem möglichst enantiomerenreinen Produkt führen, das sich dann anschließend in einfacher Weise isolieren und reinigen läßt.

Das Verfahren umfasst entweder die bisher nicht bekannte Verbindung N-Methyl-N-[(1S)-1-phenyl-2-((3S)-3-hydroxypyrrolidin-1-yl)-ethan] als neues Zwischenprodukt zur Herstellung von N-Methyl-N-[(1S)-1-phenyl-2-((3S)-3-hydroxypyrrolidin-1-yl)ethyl]-2,2-diphenylacetamid oder N-Methyl-N-[(1R)-1-phenyl-2-((3R)-3-hydroxypyrrolidin-1-yl)-ethan] als neues Zwischenprodukt zur Herstellung von N-Methyl-N-[(1 R)-1-phenyl-2-((3R)-3-hydroxypyrrolidin-1-yl)ethyl]-2,2-diphenylacetamid.

Es wurde gefunden, daß sich Verbindungen der Formel (III) 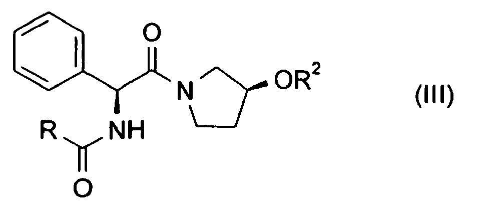 worin R und R² die folgenden Bedeutungen haben,
- R: H, OR¹ oder SR¹ ,
- R¹: A, Aryl, Heteroaryl, Si(R³)₃ oder COR³,
- R²: H, A, Aryl, Heteroaryl sowie Si(R³)₃ oder COR³,
- R³: H, A, Aryl oder Heteroaryl,
- A: geradkettiger oder verzweigter Alkylrest mit 1 bis 6 C-Atomen,
in hohen Ausbeuten und enantiomerenrein darstellen lassen, indem, je nach gewünschtem Endprodukt, (3S)-3-Hydroxypyrrolidine oder (3R)-3-Hydroxypyrrolidine der Formel (II) 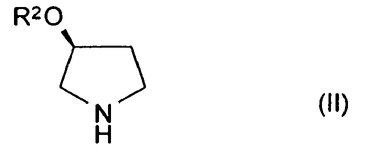 worin
- R²: H, A, Aryl, Heteroaryl sowie Si(R³)₃ oder COR³ und
- R³: H, A, Aryl oder Heteroaryl
bedeuten, oder deren Salze, gebildet mit HCl, HBr, HI, H₂SO₄, H₃PO₄ oder geeigneten organischen Säuren,
mit entsprechenden (S)- oder (R)-enantiomeren Formen von N-substituierten Phenylglycinen der Formel (I) 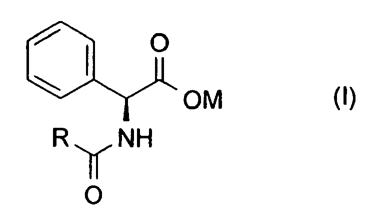 worin
- R: H, OR¹ oder SR¹,
- R¹: A, Aryl, Heteroaryl, Si(R³)₃ oder COR³,
- R³: H, A, Aryl oder Heteroaryl,
- M: H oder ein Kation aus der Gruppe Alkali, Erdalkali, Ammonium oder Alkylammonium
bedeuten,
amidisch gekuppelt werden.

Alkyl hat 1 bis 6, vorzugsweise 1, 2, 3 oder 4 C-Atome. Alkyl bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Aryl bedeutet vorzugsweise unsubstituiertes Phenyl oder ein- oder zweifach durch Hal, OA oder Alkyl substituiertes Phenyl, fermer z.B. Biphenyl oder Naphthyl.

Heteroaryl bedeutet vorzugsweise z.B. Furanyl, Thiophenyl, Pyridinyl, Pyrrolyl oder Thiazolyl.

Si(R³)₃ bedeutet vorzugsweise z.B. Si(CH₃)₃.

COR³ bedeutet vorzugsweise z.B. Acetyl oder Benzoyl.

R bedeutet vorzugsweise insbesondere z.B. Methoxy oder Ethoxy.

R¹ bedeutet insbesondere z.B. Methyl, Ethyl, Propyl, Butyl, Phenyl, Si(CH₃)₃ oder Acetyl.

R² bedeutet insbesondere z.B. H, tert.-Butyl, Si(CH₃)₃, Acetyl, Benzyl oder Benzoyl, ganz besonders bevorzugt ist H.

Die hergestellten Amide der Formel (III) lassen sich in einfacher Weise reduktiv, gegebenenfalls durch Abspaltung der Schutzgruppe der Hydroxylgruppe des Pyrrolidins in N-Methyl-N-[(1S)-1-phenyl-2-((3S)-3-hydroxypyrrolidin-1-yl)-ethan] oder N-Methyl-N-[(1R)-1-phenyl-2-((3R)-3-hydroxypyrrolidin-1-yl)-ethan] der Formel (IV) überführen.

Durch Umsetzung mit aktivierten Carbonsäuren der Formel (V) 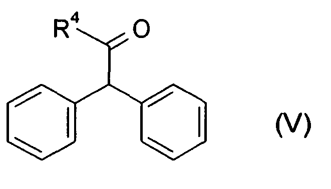 worin
- R⁴: F, Cl, Br, I, OA oder O-CO-A
bedeutet,
lassen sich aus den freien Basen der Verbindungen der Formel (IV) 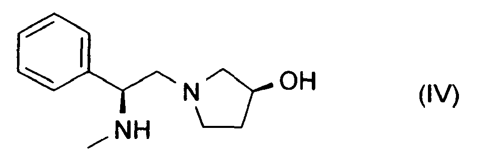 oder aus ihren Salzen, gebildet mit HCl, HBr, HI, H₂SO₄, H₃PO₄ oder geeigneten organischen Säuren die enantiomeren Verbindungen der Formel (VI) 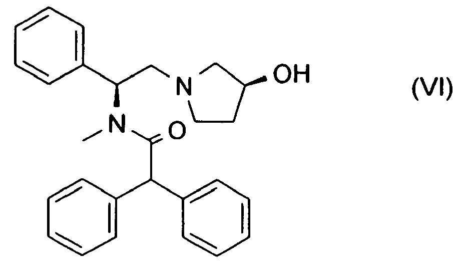 in reiner Form herstellen. Vorzugsweise werden diese Verbindungen als Hydrochloride hergestellt, wobei es sich bei der Verbindung N-Methyl-N-[(1S)-1-phenyl-2-((3S)-3-hydroxypyrrolidin-1-yl)-ethyl]-2,2-diphenylacetamid um die bekannte Form EMD 61753 handelt; aber auch die entsprechenden Salze mit den übrigen oben genannten Säuren sind analog herstellbar.

Insbesondere ist auf diese Weise durch die letzte Umsetzung mit Diphenylessigsäurechlorid N-Methyl-N-[(1S)-1-phenyl-2-((3S)-3-hydroxy-pyrrolidin-1-yl)-ethyl]-2,2-diphenylacetamid, herstellbar.

Die als Zwischenprodukt synthetisierten Verbindungen der Formel (IV) lassen sich allgemein durch Umsetzung von Verbindungen der Formel (I) mit solchen der Formel (II) gewinnen. Vorzugsweise werden in dieser Reaktion Verbindungen der Formel (I) verwendet, in denen R die Bedeutung OR¹ mit R¹ A, Aryl, Heteroaryl, Si(R³)₃ oder COR² und R² H, Alkyl, Aryl oder Heteroalkyl, mit den oben angegebenen bevorzugten Bedeutungen, besitzt. Überraschenderweise werden im Gegensatz zur Verwendung der entsprechenden Formylverbindung enantiomerenreine Reaktionsprodukte der Formel (III) erhalten. Auf diese Weise kann vorteilhafterweise die Auftrennung des Racemats entfallen.

Die Umsetzung der Verbindungen (I) und (II) kann in einem beliebigen aprotischen Lösungsmittel erfolgen. Besonders geeignet sind polare aprotische Lösungsmittel aus der Gruppe Diethylether, Petrolether, Aceton, Nitrobenzol, Dimethylformamid, Dimethylsulfoxid oder andere entsprechende Lösungsmittel. Hierbei werden die Edukte in so viel Lösungsmittel aufgenommen, daß eine 10 bis 30 prozentige Lösung erhalten wird. Bevorzugt wird die Reaktion in Tetrahydrofuran als Lösungsmittel durchgerührt.

Die Reaktionen der Verbindungen (I) und (II) erfolgen unter geeigneten Bedingungen bei Temperaturen zwischen 0 bis 50 °C. Besonders gute Ergebnisse werden jedoch bei Raumtemperaturen zwischen 20 und 30°C und bei Normaldruck erzielt.

Zur Aktivierung der Edukte ist die Gegenwart eines Hilfsreagenzes erforderlich. Dieses können Hilfsmittel sein, die auch als Peptidkupplungsreagenzien verwendet werden. Geeignet sind Verbindungen wie beispielsweise Phosphoroxytrichlorid, Phosphorhalogenide der Wertigkeit III und V, Phosgen, Dicyclohexylcarbodiimid, Tributylammoniumsalz des Pyridins, Phenyldichlorphosphat, 2-Chlor-1,2,3-trinitrobenzol, Phosphorsäureester, Chlorsulfonylisocyanat, CH₃SO₂Cl-(C₂H₅)₃N, (C₆H₅)₃P-CCl₄-(C₂H₅)₃N, N,N'-Carbonyldiimidazol" N-(Alkylcarbonyl)-imidazole, Säureanhydride oder Säurechloride und insbesondere Alkylchlorformiate, wie Chlorameisensäureethylester. Andere geeignete Hilfsreagenzien sind in verschiedenen Fachbüchern beschrieben, wie z. B. in C. Ferri "Reaktionen der organischen Synthese"; R. C. Larock "Comprehensive Organic Transformations; A Guide to Functional Group Preparations", Verlag Chemie, 1989.

Weiterhin ist die Gegenwart einer Base erforderlich. Geeignete sind ebenfalls aus den oben genannten Fachbüchern zu entnehmen. Solche Basen sind beispielsweise tertiäre Amine, wie z. B. Triethylamin. Es können aber auch anorganische Basen hinzugefügt werden. Als anorganische Basen sind insbesondere Carbonate geeignet. Bei Verwendung der Alkalihydroxide, wie NaOH oder KOH ist besonders auf eine genaue Dosierung zu achten, da es sonst zu unerwünschten Nebenreaktionen kommt. Zur Vereinfachung der Aufarbeitung ist es aber auch möglich, das Hydroxypyrrolidin im Überschuß einzusetzen, so daß es selbst als Base wirkt.

Die Aufarbeitung des erhaltenen Reaktionsprodukts (III) kann nach dem Abfiltrieren des angefallenen Niederschlags mit laborüblichen Methoden aus dem Filtrat erfolgen. Beispielsweise besteht eine gängige und geeignete Methode darin, das Lösungsmittel abzudestillieren, das Rohprodukt erneut in einem organischen Lösungsmittel aufzunehmen, die erhaltene Lösung mit Wasser mehrfach zu extrahieren, das Lösungsmittel erneut abzudestillieren und das erhaltene Produkt durch Umkristallisation aus einem geeigneten Lösungsmittel, wie z. B. aus Methanol umzukristallisieren. Es sind aber auch andere, dem Fachmann bekannte Aufarbeitungsvarianten möglich, wie z. B. solche, die eine chromatographische Aufreinigung mit einschließen.

Je nach Reaktionsbedingungen, wird das Reaktionsprodukt (III) als freie Base oder als Säureadditionssalz der Säuren HCl, HBr, HI, H₂SO₄ oder einer organischen Carbonsäure aus einem wasserhaltigen Lösungsmittelgemisch erhalten. In letzteren Fällen kann die Isolierung nach der Phasentrennung nach laborüblichen Methoden erfolgen.

Als geeignete organische Carbonsäuren können insbesondere aliphatische, alicyclische, araliphatische, aromatische oder heterocyclische ein-oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure verwendet werden.

Die Reduktion der Verbindungen der Formel (III) erfolgt unter Schutzgasatmosphäre, z. B. unter Stickstoffatmosphäre, in Gegenwart eines Hydridtransfer-Reagenzes. Geeignete Hydridtransfer-Reagenzien sind solche aus der Gruppe der Metallaluminiumhydride, vorzugsweise Lithiumaluminiumhydrid, Metall-Alkoxy-aliminiumhydride, wie z. B. Li-Triethoxyaluminiumhydrid, Metallborhydride, vorzugsweise NaBH₄, oder Boran, wobei zusätzlich die Gegenwart einer Lewissäure erforderlich ist, wie z. B. Bortrifluorid.

Die Reduktion wird vorzugsweise in einem polaren aprotischen und hydridinerten Lösungsmittel durchgeführt. Geeignet sind die gleichen, wie oben bereits genannt. Besonders geeignet sind z. B. Diethylether oder Tetrahydrofuran.

Zur Durchführung der Hydrierung wird eine Verbindung der Formel (III) in einem geeigneten Lösungsmittel gelöst und zu einer Lösung, die das Hydridtransfer-Reagenz in äquimolaren Mengen bzw. in geringem Überschuß enthält, unter Erwärmen zugegeben. Es ist aber auch möglich, die zu hydrierende Ausgangsverbindung vorzulegen und das Hydrierungsreagenz in entsprechender Menge auf geeignete Weise zuzugeben, so daß ein Reaktionsgemisch erhalten wird, worin das Edukt eine Konzentration von 10 bis 25 Gew.-% besitzt, bezogen auf das Lösungsmittel. Zur Beendigung der Reaktion wird das Reaktionsgemisch für mehrere Stunden unter Rückflußbedingungen gerührt. Die Reaktionslösung wird anschließend nach dem Fachmann bekannten Methoden aufbereitet, indem u. a. durch Zugabe eines Lösungsmittelgemisches, bestehend aus einem protonenliefernden und einem aprotischen Lösungsmittel, der Überschuß Hydridtransfer-Reagenz zersetzt und das Reaktionsprodukt freigesetzt wird. Als protonenliefernde Lösungsmittel sind z. B. Wasser oder Alkohole wie Ethanol oder Methanol geeignet. Als aprotische Lösungsmittel sind alle oben bereits genannten polaren aprotischen Lösungsmittel geeignet, insbesondere Tetrahydrofuran. Letzteres wird vorzugsweise eingesetzt, da es technisch als wasserfreies Produkt erhältlich ist.

Die Produktaufarbeitung kann nach der Phasentrennung nach laborüblichen Methoden erfolgen. Das erhaltene Rohprodukt kann durch Kristallisationsmethoden aufgearbeitet werden oder es wird zur Aufarbeitung beispielsweise in einem organischen nicht wassermischbaren Lösungsmittel aufgenommen und mit einem Überschuß einer anorganischen Säure, vorzugsweise Salzsäure, versetzt. Das auf diese Weise gebildete Salz kann anschließend kristallin abgetrennt werden.

Die weitere Umsetzung von N-Methyl-N-[(1S)-1-phenyl-2-((3S)-3-hydroxypyrrolidin-1-yl)-ethan oder dessen Dihydrochlorid mit einem geeigneten Diphenylessigsäurederivat, vorzugsweise dem Säurechlorid, zu dem gewünschten Endprodukt N-Methyl-N-[(1S)-1-phenyl-2-((3S)-3-hydroxypyrrolidin-1-yl)-ethyl]-2,2-diphenylacetamid (Formel VI, EMD 61753) erfolgt nach Methoden wie sie in DE-A1-40 34 785 und DE-A1-42 15 213 bzw. EP 0 569 802 A1 beschrieben sind.

Die vorliegende Erfindung betrifft die Verwendung von N-Methyl-N-[(1S)-1-phenyl-2-((3S)-3-hydroxypyrrolidin-1-yl)-ethyl]-2,2-diphenylacetamid und/oder eines physiologisch verträglichen Salzes davon zur Herstellung eines Arzneimittels zur Behandlung von Irritable Bowel Syndrome (IBS).

Außerdem wird ein Verfahren zur wahlweisen Herstellung von N-Methyl-N-[(1S)-1-phenyl-2-((3S)-3-hydroxypyrrolidin-1-yl)ethyl]-2,2-diphenylacetamid oder N-Methyl-N-[(1R)-1-phenyl-2-((3R)-3-hydroxypyrrolidin-1-yl)ethyl]-2,2-diphenylacetamid offenbart, das dadurch gekennzeichnet ist, daß
a) ein N-substituiertes Phenylglycinderivat der Formel I 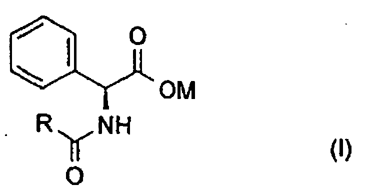 worin
   - R: H, OR¹ oder SR¹,
   - R¹: A, Aryl, Heteroaryl, Si(R³)₃ oder COR³,
   - R³: H, A, Aryl oder Heteroaryl,
   - A: geradkettiger oder verzweigter Alkylrest mit 1 bis 6 C-Atomen,
   - M: H oder ein Kation aus der Gruppe Alkali-, Erdalkali-Ammonium- oder Alkylammonium
   bedeuten,
   mit einer Verbindung der Formel II 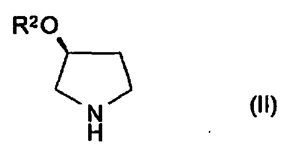 worin
   - R²: H, A, Aryl, Heteroaryl, Si(R³)₃ oder COR³
   und
   - R³: H, A, Aryl oder Heteroaryl
   bedeuten
   oder mit einem Säureadditionssalz der Verbindung der Formel (II), der Säuren HCl, HBr, HI, H₂SO₄, H₃PO₄ oder einer organischen Carbonsäure, zu einer Verbindung der Formel III 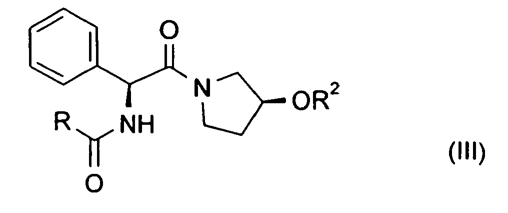 worin R und R² die oben gegebenen Bedeutungen haben,
   umgesetzt wird,
b) anschließend durch Reduktion in eine Verbindung der Formel (IV) 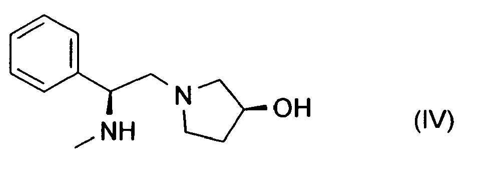 umgesetzt wird, die gegebenenfalls in ein entsprechendes Säureadditionssalze der Säuren HCl, HBr, Hl, H₂SO₄, H₃PO₄ oder in ein Salz einer organischen Carbonsäure umgewandelt wird, und
c) die so erhaltene Verbindung der Formel (IV) mit einer aktivierten Carbonsäure der Formel (V) 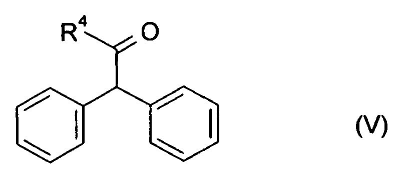 worin
   - R⁴: F, Cl, Br, I, OA oder O-CO-A,
   bedeutet,
   zu der Verbindung der Formel (VI) 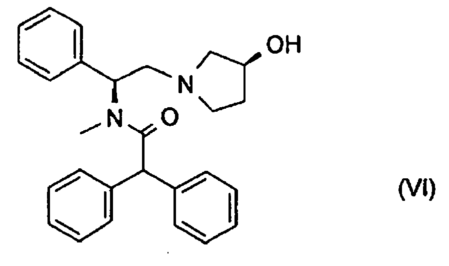 umgesetzt wird,
   die gegebenenfalls mit einer anorganischen Säure aus der Gruppe HCl, HBr, HI, Schwefelsäure, Sulfaminsäure, Salpetersäure, Phosphorsäure, Orthophosphorsäure, oder mit einer organischen Säure in das zugehörige Säureadditionssalz überführt wird,
   wobei in Stufe a) Edukte in Abhängigkeit vom als Endprodukt gewünschten Enantiomeren eingesetzt werden.

Das hier offenbarte Verfahren betrifft auch die Verbindungen
N-Methyl-N-[(1S)-1-phenyl-2-((3S)-3-hydroxypyrrolidin-1-yl)-ethan] und/oder
N-Methyl-N-[(1R)-1-phenyl-2-((3R)-3-hydroxypyrrolidin-1-yl)-ethan].

Das hier offenbarte Verfahren betrifft auch die Verbindung N-Methyl-N-[(1S)-1-phenyl-2-((3S)-3-hydroxypyrrolidin-1-yl)-ethan] als Zwischenprodukt zur Herstellung von N-Methyl-N-[(1S)-1-phenyl-2-((3S)-3-hydroxypyrrolidin-1-yl)ethyl]-2,2-diphenylacetamid.

Das hier offenbarte Verfahren betrifft auch die Verbindung N-Methyl-N-[(1R)-1-phenyl-2-((3R)-3-hydroxypyrrolidin-1-yl)-ethan] als Zwischenprodukt zur Herstellung von N-Methyl-N-[(1R)-1-phenyl-2-((3R)-3-hydroxypyrrolidin-1-yl)ethyl]-2,2-diphenylacetamid.

Offenbart wird hiermit auch ein Verfahren wie vorstehend beschrieben, das dadurch gekennzeichnet ist, daß Verbindungen der Formel (I) verwendet werden, in denen R die Bedeutung OR¹ mit R¹ A, Aryl, Heteroaryl, Si(R³)₃ oder COR³ und R³ H, A, Aryl oder Heteroalkyl besitzt.

Offenbart wird hiermit betrifft daher auch ein Verfahren wie vorstehend beschrieben, das dadurch gekennzeichnet ist, daß die Umsetzung der Verbindungen (I) und (II) in einem aprotischen, vorzugsweise polaren aprotischen Lösungsmittel bei einer Temperatur von 0 bis 50 °C, vorzugsweise bei Temperaturen zwischen 20 und 30 °C, erfolgt.

Offenbart wird hiermit auch ein Verfahren wie vorstehend beschrieben, das dadurch gekennzeichnet ist, daß die Umsetzung der Verbindungen (I) und (II) in einem Lösungsmittel aus der Gruppe Dieethylether, Petrolether, Aceton, Nitrobenzol, Dimethylformamid, Dimethylsulfoxid, Tetrahydrofuran durchgeführt wird, wobei die Edukte in dem Lösungsmittel in einer Konzentration von 10 bis 30 % vorliegen.

Offenbart wird hiermit auch ein Verfahren wie vorstehend beschrieben, das dadurch gekennzeichnet ist, daß die Umsetzung der Verbindungen (I) und (II) in Gegenwart eines Hilfsreagenzes aus der Gruppe Phosphoroxytrichlorid, Phosphorhalogenide der Wertigkeit III und V, Phosgen, Dicyclohexylcarbodiimid, Tributylammoniumsalz des Pyridins, Phenyldichlorphosphat, 2-Chlor-1,2,3-trinitrobenzol, Phosphorsäureester, Chlorsulfonylisocyanat, CH₃SO₂Cl-(C₂H₅)₃N, (C₆H₅)₃P-CCl₄-(C₂H₅)₃N, N,N'-Carbonyldiimidazol,
N-(Alkylcarbonyl)-imidazole, Acetanhydrid, Essigsäurechlorid und Chlorameisensäureethylester sowie einer organischen oder anorganischen Base erfolgt.

In einem Aspekt ist das offenbarte Verfahren wie vorstehend beschrieben, dadurch gekennzeichnet, daß die Umsetzung der Verbindungen (I) und (II) in Gegenwart einer Base aus der Gruppe Triethylamin, Natriumcarbonat, Kaliumcarbonat, Kalziumcarbonat, NaOH, KOH erfolgt.

In einem Aspekt ist das offenbarte Verfahren wie vorstehend beschrieben, dadurch gekennzeichnet, daß die Reduktion der Verbindungen der Formel (III) in Gegenwart eines Hydridtransfer-Reagenzes aus der Gruppe der Metallaluminiumhydride, vorzugsweise Lithiumaluminiumhydrid, der Metall-Alkoxy-aliminiumhydride, vorzugsweise Li-Triethoxyaluminiumhydrid, der Metallborhydride, vorzugsweise NaBH₄, oder Boran, sowie gegebenenfalls in Gegenwart einer Lewissäure, wie Bortrifluorid, in einem polaren aprotischen Lösungsmittel aus der Gruppe Dieethylether, Petrolether, Aceton, Nitrobenzol, Dimethylformamid, Dimethylsulfoxid, Tetrahydrofuran erfolgt.

In einem Aspekt ist das offenbarte Verfahren wie vorstehend beschrieben, dadurch gekennzeichnet, daß eine Verbindung der Formel (III) als Edukt in einem Lösungsmittel in einer Konzentration von 10 bis 25 % gelöst wird und das Hydrierungsprodukt durch Zugabe eines protonenliefernden Lösungsmittels im Gemisch mit einem aprotischen Lösungsmittel freigesetzt wird.

Die im nachfolgenden gegebenen Beispiele werden zur Veranschaulichung der vorliegenden Erfindung gegeben, können aber nicht dazu dienen, die beanspruchte Erfindung auf diese zu beschränken.

### BEISPIELE

N-substituierte (2S)-2-Phenylglycin-N,N-[(3S)-3-hydroxy-tetramethylen-amide] der Formel III aus (2S)-2-Phenylglycinen der Formel I

### Beispiel 1

### (2S)-N-Formyl-2-phenylglycin-N,N-[(3S)-3-hydroxy-tetramethylenamid]

Aus (2S)-N-Formyl-2-phenylglycin (erhältlich aus (S)-(+)-alpha-Amino-phenylessigsäure und Acetanhydrid / Ameisensäure z.B. nach Huszthy, Peter; Oue, Masatoshi; Bradshaw, Jerald S.; Zhu, Cheng Y.; Wang, Tingmin; et al., J.Org.Chem., EN, 57 (20) [1992] 5383-5394)
und

(3S)-3-Hydroxypyrrolidin (erhältlich aus kommerziellem (S)-1-Benzyl-3-pyrrolidinol z.B. nach Bhat, Krishna L.; Flanagan, Denise M.; Joullie, Madeleine M., Synth.Commun., EN, 15 (7) [1985] 587-598 oder Naylor, Alan; Judd, Duncan B.; Scopes, David I. C.; Hayes, Ann G.; Birch, Philip J., J.Med.Chem., EN, 37 (14) [1994] 2138-2144):

Unter Stickstoffatmosphäre werden zu 9 g (2S)-N-Formyl-2-phenylglycin und 5,5 ml N-Methylmorpholin in 250 mL THF bei -15°C 4,8 ml Ethylchlorformiat in 10 mL Tetrahydrofuran unter Rühren zugesetzt und nach 10 min Wartezeit eine Lösung von 6,2 g (3S)-3-Hydroxypyrrolidin-Hydrochlorid und 7 mL Triethylamin in 50 mL Dimethylformamid. Nach 18 Stunden Rühren wird der angefallene Niederschlag abgetrennt und entstandenes (2S)-N-Formyl-2-phenylglycin-N,N-[(3S)-3-hydroxy-tetramethylenamid mit laborüblichen Methoden durch Aufkonzentrieren, und anschließende chromatographische Reinigung aus dem Filtrat isoliert.
1 H-NMR: D₆-DMSO; 3,0-3,8 (m), 4,25 (d), 5,0 (s,br), 5,7 (dd), 7,4 (ArH), 8,0 (ArH), 8,8 (CHO):
MS-FAB: (M+1)⁺ 221, 205;
Kristalle Fp.: 97-101°C;
[α]_{D}²⁰ = +208, c =1 in Methanol.

### Beispiel 2

### (2S)-N-Carboxybenzyl-2-phenylglycin-N,N-[(3S)-3-hydroxytetramethylenamid]

Aus (2S)-N-Carboxybenzyl-2-phenylglycin (aus (S)-(+)-alpha-Aminophenylessigsäure und Chlorkohlensäurebenzylester z.B. nach Jones, Raymond CF; Turner, lan; Howard, Kevin J., Tetrahedron Lett., 34 (39) [1993] 6329-6332)
und

(3S)-3-Hydroxypyrrolidin (erhältlich aus kommerziellem (S)-1-Benzyl-3-pyrrolidinol z.B. nach Bhat, Krishna L.; Flanagan, Denise M.; Joullie, Madeleine M., Synth.Commun., EN, 15 (7) [1985] 587-598 oder Naylor, Alan; Judd, Duncan B.; Scopes, David I. C.; Hayes, Ann G.; Birch, Philip J., J.Med.Chem., EN, 37 (14) [1994] 2138-2144):

Unter Stickstoffatmosphäre werden 14,3 g (2S)-N-Carboxy-benzyl-2-phenylglycin in 100 ml Tetrahydrofuran in der Kälte mit 5,5 ml 4-Methylmorpholin und einer Lösung aus 4,8 ml Ethylchlorformiat und 10 ml Tetrahydrofuran versetzt und dann 30 min gerührt. Dann wird eine Lösung aus 4,36 g (3S)-3-Hydroxypyrrolidin und 10 ml Tetrahydrofuran zugeben. Nach 18 Stunden Rühren wird der angefallene Niederschlag abgetrennt und das gebildete (2S)-N-Carboxybenzyl-2-phenylglycin-N,N-[(3S)-3-hydroxy-tetramethylenamid mit laborüblichen Methoden durch Aufkonzentrieren, Aufnehmen in einem organischen Solvens, Waschen mit einer wäßrigen Phase, erneutes Aufkonzentrieren und Kristallisation aus dem Filtrat isoliert.
1H-NMR: D₆-DMSO+TFA; 5,1 (s), PhCH₂R;
FAB-MS: 355 (M+1)⁺, 311, 196, 176;
Konsistenz: Öl;
[α]_{D}²⁰ = + 108, c=1 in Methanol.

### Beispiel 3

### (2S)-N-Carboxyethyl-2-phenylglycin-N,N-[(3S)-3-hydroxy-tetramethylenamid]

### 3.a)

Aus (2S)-N-Carboxyethyl-2-phenylglycin (aus (S)-(+)-alpha-Amino-phenylessigsäure und Chlorkohlensäureethylester z.B. nach Bodurow, C. C.; Boyer, B. D.; Brennan, J.; Bunnell, C. A.; Burks, J. E.; et al., Tetrahedron Lett., EN, 30 (18) [1989] 2321-2324)
und

(3S)-3-Hydroxypyrrolidin (erhältlich aus kommerziellem (S)-1-Benzyl-3-pyrrolidinol z.B. nach Bhat, Krishna L.; Flanagan, Denise M.; Joullie, Madeleine M., Synth.Commun., EN, 15 (7) [1985] 587-598 oder Naylor, Alan; Judd, Duncan B.; Scopes, David I. C.; Hayes, Ann G.; Birch, Philip J., J.Med.Chem., EN, 37 (14) [1994] 2138-2144)

Unter Stickstoffatmosphäre werden 16,7 g (2S)-N-Carboxyethyl-2-phenylglycin in 100 ml Tetrahydrofuran in der Kälte mit 8,3 ml 4-Methylmorpholin und einer Lösung aus 7,1 ml Ethylchlorformiat und 20 ml Tetrahydrofuran versetzt und dann 60 min gerührt. Dann wird eine Lösung aus 6,5 g (3S)-3-Hydroxypyrrolidin und 30 ml Tetrahydrofuran zugeben. Nach 18 Stunden Rühren wird der angefallene Niederschlag abgetrennt und das (2S)-N-Carboxyethyl-2-phenylglycin-N,N-[(3S)-3-hydroxy-tetramethylenamid mit laborüblichen Methoden durch Aufkonzentrieren, Aufnehmen in einem organischen Solvens, Waschen mit einer wäßrigen Phase, erneutes Aufkonzentrieren und Kristallisation aus dem Filtrat isoliert.

### 3.b)

Aus (2S)-N-Carboxyethyl-2-phenylglycin (s.o.) und (3S)-3-Hydroxy-pyrrolidin-Hydrochlorid (kommerziell erhältlich): Zu 11 g Ethylchlorformiat in 100 ml THF werden bei ca - 10 °C eine Mischung von 24 g (2S)-N-Carboxyethyl-2-phenylglycin mit 10 g Methylmorpholin in 100 ml THF dosiert. Es folgt nach einer Nachrührphase eine Mischung von 12 g (3S)-3-Hydroxypyrrolidin-Hydrochlorid in 10 ml VE-Wasser sowie eine Mischung von 10 g Methylmorpholin in 20 ml THF. Nach mehrstündigem Nachrühren und Phasentrennung wird das (2S)-N-Carboxyethyl-2-phenylglycin-N,N-[(3S)-3-hydroxy-tetramethylenamid mit laborüblichen Methoden durch Aufkonzentrieren, Aufnehmen in einem organischen Solvens, Waschen mit einer wäßrigen Phase, erneutes Aufkonzentrieren und Kristallisation isoliert.

Die analytischen Daten zu den Varianten 3a und 3b entsprechen sich:
1H-NMR: D₆-DMSO; 1,2 (t), 3 - 3,8 (m, br), 4,05 (q), 4,25 (s, br), 7,25-7,45 (m);
MS: 293 (M+1)⁺, 247, 178, 106;
Kristalle Fp.: 124-126°C;
[α]_{D}²⁰ = + 137 C = 1 in Methanol.

### N-Methyl-N-[(1S)-1-phenyl-2-((3S)-3-hydroxypyrrolidin-1-yl)-ethane der Formel IV

### Beispiel 4

### N-Methyl-N-[(1S)-1-phenyl-2-((3S)-3-hydroxypyrrolidin-1-yl)-ethan = 1-[(3S)-3-Hydroxypyrrolidin-1-yl]-(2S)-2-methyl-amino-2-phenylethan

Unter Stickstoff werden 2200 ml einer 1,08 molaren Lithiumaluminiumhydrid-Tetrahydrofuran-Lösung leicht erwärmt und unter Rühren eine Lösung aus 264 g (2S)-N-Carboxyethyl-2-phenylglycin-N,N-[(3S)-3-hydroxy-tetramethylenamid] und 1400 ml Tetrahydrofuran zudosiert. Nach dem Dosierungsende wird 3 Stunden refluxiert und die erkaltete Reaktionslösung mittels einer Wasser/Tetrahydrofuran-Mischung hydrolysiert. Nach Natriumcarbonat-Behandlung und Abtrennung anorganische Bestandteile wird das Produkt mit laborüblichen Methoden aus dem Filtrat isoliert. Das ölige Rohprodukt bildet nach Aufreinigung mittels Kristallisation oder Chromatographie einen Feststoff.
1H-NMR: D₆-DMSO; 2,1-3,1(m), 3,6(dd), 4,3(m), 7,15-7,35 (m);
MS: 220 (M⁺), 205, 120, 100, 91;
Aussehen: gelbliches Öl, welches chargenabh. durchkristallisiert;
[α]_{D}²⁰ = + 66,8; c = 0,0938 g in 10 ml Methanol.

### Beispiel 5

### N-Methyl-N-[(1S)-1-phenyl-2-((3S)-3-hydroxypyrrolidin-1-yl)-ethan-dihydrochlorid =

### 1-[(3S)-3-Hydroxypyrrolidin-1-yl]-(2S)-2-methyl-amino-2-phenylethan Dihydrochlorid

Unter Stickstoff werden 2200 ml einer 1,08 molaren Lithiumaluminiumhydrid-Tetrahydrofuran-Lösung leicht erwärmt und unter Rühren eine Lösung aus 264 g (2S)-N-Carboxyethyl-2-phenylglycin-N,N-[(3S)-3-hydroxy-tetramethylenamid] und 1400 mL Tetrahydrofuran zudosiert. Nach dem Dosierungsende wird noch 3 Stunden refluxiert, dann abgekühlt und die Reaktionslösung mittels einer Mischung aus 80 ml Wasser und 400 ml Tetrahydrofuran hydrolysiert. Nach Natriumcarbonat-Behandlung und Abtrennung anorganische Bestandteile wird das Produkt mit laborüblichen Methoden aus dem Filtrat isoliert. Das ölige Rohprodukt wird in einem organischen, nicht wassermischbarem Lösungsmittel aufgenommen und mit einem Überschuß Salzsäure versetzt. Das kristalline Produkt wird isoliert und getrocknet.
1H-NMR: D₆-DMSO; 3,4(m), 3,8(m), 4,2(m), 4,4(m), 4,9(m), 7,5 u. 7,8 (ArH);
Schmelzpunkt: 240-242°C;
[α]_{D}²⁰ = -22,4; c=1 in Wasser.

## Patentansprüche

1. Verwendung von N-Methyl-N-[(1S)-1-phenyl-2-((3S)-3-hydroxypyrrolidin-1-yl)-ethyl]-2,2-diphenylacetamid und/oder eines physiologisch verträglichen Salzes davon zur Herstellung eines Arzneimittels zur Behandlung von Irritable Bowel Syndrome (IBS).

2. Verwendung gemäß Anspruch 1, wobei das physiologisch verträgliche Salz von N-Methyl-N-[(1S)-1-phenyl-2-((3S)-3-hydroxypyrrolidin-1-yl)-ethyl]-2,2-diphenylacetamid das Hydrochloridsalz ist.

## Claims

1. Use of N-methyl-N-[(1S)-1-phenyl-2-((3S)-3-hydroxypyrrolidin-1-yl)-ethyl]-2,2-diphenylacetamide and/or a physiologically acceptable salt thereof for the manufacture of a medicament for the treatment of Irritable Bowel Syndrome (IBS).

2. Use according to claim 1, wherein the physiologically acceptable salt of N-methyl-N-[(1S)-1-phenyl-2-((3S)-3-hydroxypyrrolidin-1-yl)-ethyl]-2,2-diphenylacetamide is the hydrochloride salt.

## Revendications

1. Utilisation de N-méthyl-N-[(1S)-1-phényl-2-((3S)-3-hydroxypyrrolidine-1-yl)-éthyl]-2,2-diphénylacétamide et/ou d'un sel physiologiquement acceptable pour fabriquer un médicament destiné à traiter le syndrome du côlon irritable (IBS).

2. Utilisation selon la revendication 1, dans laquelle le sel physiologiquement acceptable de N-méthyl-N-[(1S)-1-phényl-2-((3S)-3-hydroxypyrrolidine-1-yl)-éthyl]-2,2-diphénylacétamide est le sel d'hydrochlorure.
